# EUROPÄISCHE PATENTSCHRIFT

(11) **EP 1 905 835 B1**
(45) Veröffentlichungstag und Bekanntmachung des Hinweises auf die Patenterteilung: **06.06.2012**
(21) Anmeldenummer: 07115590.7
(22) Anmeldetag: 04.09.2007
(51) Int. Cl.: C12N 15/62

(54) **Signalpeptid zur Produktion von rekombinanten Proteinen**
Signal peptide for the production of recombinant proteins
Peptide signal pour la production de protéines recombinantes

(30) Priorität: 22.09.2006 DE 102006044841
(43) Veröffentlichungstag der Anmeldung: 02.04.2008
(73) Patentinhaber: Wacker Chemie AG, 81737 München (DE)
(72) Erfinder: Leonhartsberger, Susanne, Dr., 07743 Jena (DE); Candussio, Anton, Dr., 81825 München (DE); Schmid, Gerhard, Dr., 82131 Gauting (DE)
(74) Vertreter: Potten, Holger

(56) Entgegenhaltungen:
- WO-A-01/21662
- WO-A-01/94418
- CHOI J H ET AL: "Secretory and extracellular production of recombinant proteins using Escherichia coli" APPLIED MICROBIOLOGY AND BIOTECHNOLOGY, SPRINGER VERLAG, BERLIN, DE, Bd. 64, Nr. 5, 14. Februar 2004 (2004-02-14), Seiten 625-635, XP002402621 ISSN: 0175-7598

### SEQUENZPROTOKOLL

<110> Consortium fuer elektrochemische Industrie GmbH
<120> Signalpeptid zur Produktion von rekombinanten Proteinen
<130> Co10612
<140>
   <141>
<160> 13
<170> PatentIn Vers. 2.0
<210> 1
   <211> 30
   <212> PRT
   <213> Artificial Sequence
<220>
   <223> Aminosaeure-Sequenz des "AFA"-Signalpeptids
<400> 1
   Met Lys Arg Asn Arg Phe Phe Asn Thr Ser Ala Ala Ile Ala Ile Ser
   1 5 10 15
   Ile Ala Leu Gln Ile Phe Phe Pro Ser Ala Ser Ala Phe Ala
   20 25 30
<210> 2
   <211> 90
   <212> DNA
   <213> Artificial Sequence
<220>
   <223> DNA-Sequenz des "AFA"-Signalpeptids
<400> 2
   atgaaaagaa accgtttttt taatacctcg gctgctattg ccatttcgat tgcattacag 60
   atcttttttc cgtccgcttc cgctttcgct 90
<210> 3
   <211> 30
   <212> PRT
   <213> Klebsiella spec.
<220>
   <223> Aminosaeure-Sequenz des "cgt"-Signalpeptids
<400> 3
   Met Lys Arg Asn Arg Phe Phe Asn Thr Ser Ala Ala Ile Ala Ile Ser
   1 5 10 15
   Ile Ala Leu Asn Thr Phe Phe Cys Ser Met Gln Thr Ile Ala
   20 25 30
<210> 4
   <211> 2065
   <212> DNA
   <213> Artificial Sequence
<220>
   <223> DNS-Molekuel, welches das CGTase-Gen von Klebsiella pneumoniae enthaelt
<220>
   <221> sig_peptide
   <222> (4)..(93)
   <223> Signalsequenz des CGTase-Gens
<220>
   <221> allele
   <222> (99)..(1971)
   <223> Strukturgen des CGTase-Gens
<400> 4
   ataatgaaaa gaaaccgttt ttttaatacc tcggctgcta ttgccatttc gattgcatta 60
   aatacttttt tttgtagcat gcagacgatt gctgctgaac cagaagaaac ttatcttgat 120
   tttcgtaagg agacgatata ttttctattc cttgatcgtt tcagcgatgg agatccaagt 180
   aataatgcag ggtttaattc tgcaacctac gatcctaata atttaaaaaa atatactgga 240
   ggagatctcc gggggttgat taataaacta ccctatttaa aatcacttgg tgttacttca 300
   atctggatta ctcccccaat cgataatgtg aataatactg atgctgctgg caatactgga 360
   tatcatggtt attggggaag agattatttt cgtatagatg aacattttgg caatctcgat 420
   gatttcaaag aactgactag tttgatgcat agtcctgatt ataatatgaa actggttctt 480
   gattatgccc ctaatcattc gaatgctaat gatgaaaatg aatttggtgc actatatcgt 540
   gatggtgtgt ttattactga ttatcctacg aatgttgccg ccaatacggg ctggtatcat 600
   cacaatggtg gggtaacgaa ctggaatgat ttcttccaag tgaagaatca taatctattc 660
   aatctatcag acctcaatca atccaatact gatgtctacc agtacttgtt ggatggttct 720
   aaattttgga tcgatgctgg tgtggatgct atcaggattg atgccatcaa gcatatggac 780
   aagtctttta tacagaaatg gaccagcgat atttatgatt acagtaagtc tatcggccgg 840
   gaaggatttt ttttcttcgg tgaatggttt ggtgccagtg cgaatactac aacaggtgtt 900
   gatggtaatg ctatcgatta cgccaacact tccgggtcag cgttgctgga ttttggattc 960
   cgcgatactt tagaaagagt tttggtagga cgtagcggaa atacaatgaa aacgttaaat 1020
   agttatctga taaaaagaca aacagtcttt accagtgatg actggcaggt tgtttttatg 1080
   gataaccatg atatggcacg cattggtacc gctctgcgtt caaacgccac tacttttggt 1140
   cctggaaata atgaaaccgg tggaagtcag agtgaagctt ttgctcagaa acgtatagac 1200
   ctcggtctgg ttgcgacaat gactgtacgt ggtattcctg ccatttatta tggtactgaa 1260
   cattatgccg ctaactttac ctctaacagt tttggtcaag ttggcagtga tccttacaac 1320
   cgagagaaaa tgccaggatt tgatacggaa agtgaggctt tctccattat taaaacactg 1380
   ggtgacctaa ggaaaagtag cccggcaatt caaaatggaa cttatactga actatgggtt 1440
   aatgatgata tattagtatt tgagcggcgt tctgggaacg atattgttat tgttgcactt 1500
   aatcgtggtg aggctaacac aattaatgtt aaaaatatag cggttcctaa tggggtatat 1560
   ccgagtttga ttgggaataa tagtgtttca gtagcaaata aacggacaac actaacactt 1620
   atgcaaaatg aagctgttgt cattcgctca caatcagatg atgcggagaa ccctacagta 1680
   caaagcataa acttcacatg taataacggt tatacgattt caggtcaaag tgtttatatt 1740
   attggtaata tacctcagtt aggtggttgg gacttaacta aagcggtaaa aatatcaccg 1800
   acacaatatc cacaatggag tgcgagctta gagcttcctt ctgacttaaa tgttgaatgg 1860
   aagtgtgtga aacgtaatga aaccaatccg acggctaatg ttgagtggca gtctggtgca 1920
   aataaccagt tcaatagcaa tgacacacaa acaacgaatg gctcgtttta attaaaattt 1980
   agtggaccag cgttccaatc gatggtccac tattcgtact ccggccataa ttatttttga 2040
   ctaatactct tacaaatttt caacc 2065
<210> 5
   <211> 700
   <212> DNA
   <213> Artificial Sequence
<220>
   <223> DNA-Sequenz einer phoA-IFNalpha2b-Genfusion
<400> 5
   attctgaaat gagctgttga caattaatca tcggctcgta taatgtgtgg aattgtgagc 60
   ggataacaat ttcacacagg aaacagaatt ctaaggagga aattatatga aacaaagcac 120
   tattgcactg gcactcttac cgttactgtt tacccctgtg acaaaagctt gtgacttacc 180
   tcagacccat tcactgggct cacgccgtac gctgatgctg ttagcacaga tgcgtcgcat 240
   ttctctgttt agttgtttga aagaccgtca tgattttggg ttcccgcaag aagagtttgg 300
   taatcagttt cagaaagccg aaactattcc ggttctgcac gaaatgattc aacagatttt 360
   taacctgttt tcgacaaagg atagctctgc cgcgtgggat gaaaccttac tggataagtt 420
   ctacaccgaa ctgtaccagc aactgaatga tctggaagca tgcgttatcc agggcgtggg 480
   tgtcacagaa actccgctga tgaaggagga cagcattctg gcggtgcgca aatatttcca 540
   gcgtatcacg ctgtatctga aagagaaaaa atattcgcca tgcgcgtggg aggtcgtgcg 600
   cgcggagatc atgcgcagtt tctctttgag caccaacctg caagaatcct tgcgttccaa 660
   agaataatag tctagaagct tggctgtttt ggcggatgag                       700
<210> 6
   <211> 700
   <212> DNA
   <213> Artificial Sequence
<220>
   <223> DNA-Sequenz einer ompA-IFNalpha2b-Genfusion
<400> 6
   attctgaaat gagctgttga caattaatca tcggctcgta taatgtgtgg aattgtgagc 60
   ggataacaat ttcacacagg aaacagaatt ctaaggagga aattatatga aaaagacagc 120
   tatcgcgatt gcagtggcac tggctggttt cgctaccgta gcgcaggctt gtgacttacc 180
   tcagacccat tcactgggct cacgccgtac gctgatgctg ttagcacaga tgcgtcgcat 240
   ttctctgttt agttgtttga aagaccgtca tgattttggg ttcccgcaag aagagtttgg 300
   taatcagttt cagaaagccg aaactattcc ggttctgcac gaaatgattc aacagatttt 360
   taacctgttt tcgacaaagg atagctctgc cgcgtgggat gaaaccttac tggataagtt 420
   ctacaccgaa ctgtaccagc aactgaatga tctggaagca tgcgttatcc agggcgtggg 480
   tgtcacagaa actccgctga tgaaggagga cagcattctg gcggtgcgca aatatttcca 540
   gcgtatcacg ctgtatctga aagagaaaaa atattcgcca tgcgcgtggg aggtcgtgcg 600
   cgcggagatc atgcgcagtt tctctttgag caccaacctg caagaatcct tgcgttccaa 660
   agaataatag tctagaagct tggctgtttt ggcggatgag 700
<210> 7
   <211> 727
   <212> DNA
   <213> Artificial Sequence
<220>
   <223> DNA-Sequenz einer cgt-IFNalpha2b-Genfusion
<400> 7
   attctgaaat gagctgttga caattaatca tcggctcgta taatgtgtgg aattgtgagc 60
   ggataacaat ttcacacagg aaacagaatt ctaaggagga aattatatga aaagaaaccg 120
   tttttttaat acctcggctg ctattgccat ttcgattgca ttaaatactt ttttttgtag 180
   catgcagacg attgcttgtg acttacctca gacccattca ctgggctcac gccgtacgct 240
   gatgctgtta gcacagatgc gtcgcatttc tctgtttagt tgtttgaaag accgtcatga 300
   ttttgggttc ccgcaagaag agtttggtaa tcagtttcag aaagccgaaa ctattccggt 360
   tctgcacgaa atgattcaac agatttttaa cctgttttcg acaaaggata gctctgccgc 420
   gtgggatgaa accttactgg ataagttcta caccgaactg taccagcaac tgaatgatct 480
   ggaagcatgc gttatccagg gcgtgggtgt cacagaaact ccgctgatga aggaggacag 540
   cattctggcg gtgcgcaaat atttccagcg tatcacgctg tatctgaaag agaaaaaata 600
   ttcgccatgc gcgtgggagg tcgtgcgcgc ggagatcatg cgcagtttct ctttgagcac 660
   caacctgcaa gaatccttgc gttccaaaga ataatagtct agaagcttgg ctgttttggc 720
   ggatgag 727
<210> 8
   <211> 727
   <212> DNA
   <213> Artificial Sequence
<220>
   <223> DNA-Sequenz einer AFA-IFNalpha2b-Genfusion
<400> 8
   attctgaaat gagctgttga caattaatca tcggctcgta taatgtgtgg aattgtgagc 60
   ggataacaat ttcacacagg aaacagaatt ctaaggagga aattatatga aaagaaaccg 120
   tttttttaat acctcggctg ctattgccat ttcgattgca ttacagatct tttttccgtc 180
   cgcttccgct ttcgcttgtg acttacctca gacccattca ctgggctcac gccgtacgct 240
   gatgctgtta gcacagatgc gtcgcatttc tctgtttagt tgtttgaaag accgtcatga 300
   ttttgggttc ccgcaagaag agtttggtaa tcagtttcag aaagccgaaa ctattccggt 360
   tctgcacgaa atgattcaac agatttttaa cctgttttcg acaaaggata gctctgccgc 420
   gtgggatgaa accttactgg ataagttcta caccgaactg taccagcaac tgaatgatct 480
   ggaagcatgc gttatccagg gcgtgggtgt cacagaaact ccgctgatga aggaggacag 540
   cattctggcg gtgcgcaaat atttccagcg tatcacgctg tatctgaaag agaaaaaata 600
   ttcgccatgc gcgtgggagg tcgtgcgcgc ggagatcatg cgcagtttct ctttgagcac 660
   caacctgcaa gaatccttgc gttccaaaga ataatagtct agaagcttgg ctgttttggc 720
   ggatgag 727
<210> 9
   <211> 21
   <212> PRT
   <213> Escherichia coli
<220>
   <223> Aminosaeure-Sequenz des phoA-Signalpeptids von Escherichia coli
<400> 9
   Met Lys Gln Ser Thr Ile Ala Leu Ala Leu Leu Pro Leu Leu Phe Thr
   1 5 10 15
   Pro Val Thr Lys Ala
   20
<210> 10
   <211> 21
   <212> PRT
   <213> Escherichia coli
<220>
   <223> Aminosaeure-Sequenz des ompA-Signalpeptids von Escherichia coli
<400> 10
   Met Lys Lys Thr Ala Ile Ala Ile Ala Val Ala Leu Ala Gly Phe Ala
   1 5 10 15
   Thr Val Ala Gln Ala
   20
<210> 11
   <211> 329
   <212> DNA
   <213> Artificial Sequence
<220>
   <223> artefizielles DNA-Fragment, welches die Sequenz des AFA-Signalpeptids enthaelt
<400> 11
   attctgaaat gagctgttga caattaatca tcggctcgta taatgtgtgg aattgtgagc 60
   ggataacaat ttcacacagg aaacagataa tgaaaagaaa ccgttttttt aatacctcgg 120
   ctgctattgc catttcgatt gcattacaga tctttttccc gtccgcttcc gctttcgctg 180
   ctgaaccaga agaaacttat cttgattttc gtaaggagac gatatatttt ctattccttg 240
   atcgtttcag cgatggagat ccaagtaata atgcagggtt taattctgca acctacgatc 300
   ctaataattt aaaaaaatat actggagga 329
<210> 12
   <211> 769
   <212> DNA
   <213> Artificial Sequence
<220>
   <223> DNS-Molekuel, welches das Gen der schweren Kette des Anti-Lysozym-Fab-Fragments enthaelt
<220>
   <221> sig_peptide
   <222> (24) .. (86)
   <223> ompA-Signalpeptid
<220>
   <221> allele
   <222> (87)..(758)
   <223> Gen der schweren Kette des Anti-Lysozym-Fab-Fragments inkl. HisTag (bp 738-755)
<400> 12
   cagaattcta aggaggaaat tatatgaaaa agacagctat cgcgattgca gtggcactgg 60
   ctggtttcgc taccgtagcg caggctgaag ttaaactgca agaatccggt ccgggtctgg 120
   ttgctccgtc ccagtccctg tccatcacct gcaccgtttc cggtttctcc ctgaccggtt 180
   acggtgttaa ctgggttcgt cagccgccgg gtaaaggtct ggaatggctg ggtatgatct 240
   ggggtgacgg taacaccgac tacaactccg ctctgaaatc ccgtctgtcc atctccaaag 300
   acaactccaa atcccaggtt ttcctgaaaa tgaactccct gcacaccgac gacaccgctc 360
   gttactactg cgctcgtgaa cgtgactacc gtctggacta ctggggtcag ggtaccaccg 420
   ttaccgtttc ctccgctaaa accaccccgc cgtccgttta cccgctggct ccgggttccg 480
   ctgctcagac caactctatg gttaccctgg gttgcctggt taaaggttac ttcccggaac 540
   cggttaccgt tacctggaac tccggttccc tgtcctccgg ttgccacacc ttcccggctg 600
   ttctgcaatc cgacctgtac accctgtcct cctccgttac cgttccgtcc tccacctggc 660
   cgtccgaaac cgttacctgc aacgttgctc acccggcttc ctccaccaaa gttgacaaaa 720
   aaatcgttcc gcgtgaccat caccaccatc accattaata actgcagaa 769
<210> 13
   <211> 768
   <212> DNA
   <213> Artificial Sequence
<220>
   <223> DNS-Molekuel, welches das Gen der leichten Kette des Anti-Lysozym-Fab-Fragemnets enthaelt
<220>
   <221> sig_peptide
   <222> (26)..(115)
   <223> CGTase-Signalsequenz
<220>
   <221> allele
   <222> (116)..(757)
   <223> Gen der leichten Kette des Anti-Lysozym-Fab-Fragments
<400> 13
   aactgcagta catggagaaa ataaaatgaa aagaaaccgt ttttttaata cctcggctgc 60
   tattgccatt tcgattgcat taaatacttt tttttgtagc atgcagacga ttgctgacat 120
   cgaactgacc cagtccccgg cttccctgtc cgcttccgtt ggtgaaaccg ttaccatcac 180
   ctgccgtgct tccggtaaca tccacaacta cctggcttgg taccagcaga aacagggtaa 240
   atccccgcag ctgctggttt actacaccac caccctggct gacggtgttc cgtcccgttt 300
   ctccggttcc ggttccggta cccagtactc cctgaaaatc aactccctgc aaccggaaga 360
   cttcggttcc tactactgcc agcacttctg gtccaccccg cgtaccttcg gtggtggtac 420
   caaactggaa ctgaaacgtg ctgacgctgc tccgaccgtt tccatcttcc cgccgtcctc 480
   cgaacagctg acctccggtg gtgcttccgt tgtttgcttc ctgaacaact tctacccgaa 540
   agacatcaac gttaaatgga aaatcgacgg ttccgaacgt cagaacggtg ttctgaactc 600
   ctggaccgac caggactcca aagactccac ctactccatg tcctccaccc tgaccctgac 660
   caaagacgaa tacgaacgtc acaactccta cacctgcgaa gctacccaca aaacctccac 720
   ctccccgatc gttaaatcct tcaaccgtaa cgaataatag ctgcagaa              768

### SEQUENZPROTOKOLL

<110> Consortium fuer elektrochemische Industrie GmbH
<120> Signalpeptid zur Produktion von rekombinanten Proteinen
<130> Co10612
<140>
   <141>
<160> 13
<170> PatentIn Vers. 2.0
<210> 1
   <211> 30
   <212> PRT
   <213> Artificial Sequence
<220>
   <223> Aminosaeure-Sequenz des "AFA"-Signalpeptids
<400> 1
   Met Lys Arg Asn Arg Phe Phe Asn Thr Ser Ala Ala Ile Ala Ile Ser
   1               5                   10                  15
   Ile Ala Leu Gln Ile Phe Phe Pro Ser Ala Ser Ala Phe Ala
               20                  25                  30
<210> 2
   <211> 90
   <212> DNA
   <213> Artificial Sequence
<220>
   <223> DNA-Sequenz des "AFA"-Signalpeptids
<400> 2
   atgaaaagaa accgtttttt taatacctcg gctgctattg ccatttcgat tgcattacag 60
   atcttttttc cgtccgcttc cgctttcgct                                   90
<210> 3
   <211> 30
   <212> PRT
   <213> Klebsiella spec.
<220>
   <223> Aminosaeure-Sequenz des "cgt"-Signalpeptids
<400> 3
   Met Lys Arg Asn Arg Phe Phe Asn Thr Ser Ala Ala Ile Ala Ile Ser
   1               5                   10                  15
   Ile Ala Leu Asn Thr Phe Phe Cys Ser Met Gln Thr Ile Ala
               20                  25                  30
<210> 4
   <211> 2065
   <212> DNA
   <213> Artificial Sequence
<220>
   <223> DNS-Molekuel, welches das CGTase-Gen von Klebsiella pneumoniae enthaelt
<220>
   <221> sig_peptide
   <222> (4)..(93)
   <223> Signalsequenz des CGTase-Gens
<220>
   <221> allele
   <222> (94)..(1971)
   <223> Strukturgen des CGTase-Gens
<400> 4
   ataatgaaaa gaaaccgttt ttttaatacc tcggctgcta ttgccatttc gattgcatta 60
   aatacttttt tttgtagcat gcagacgatt gctgctgaac cagaagaaac ttatcttgat 120
   tttcgtaagg agacgatata ttttctattc cttgatcgtt tcagcgatgg agatccaagt 180
   aataatgcag ggtttaattc tgcaacctac gatcctaata atttaaaaaa atatactgga 240
   ggagatctcc gggggttgat taataaacta ccctatttaa aatcacttgg tgttacttca 300
   atctggatta ctcccccaat cgataatgtg aataatactg atgctgctgg caatactgga 360
   tatcatggtt attggggaag agattatttt cgtatagatg aacattttgg caatctcgat 420
   gatttcaaag aactgactag tttgatgcat agtcctgatt ataatatgaa actggttctt 480
   gattatgccc ctaatcattc gaatgctaat gatgaaaatg aatttggtgc actatatcgt 540
   gatggtgtgt ttattactga ttatcctacg aatgttgccg ccaatacggg ctggtatcat 600
   cacaatggtg gggtaacgaa ctggaatgat ttcttccaag tgaagaatca taatctattc 660
   aatctatcag acctcaatca atccaatact gatgtctacc agtacttgtt ggatggttct 720
   aaattttgga tcgatgctgg tgtggatgct atcaggattg atgccatcaa gcatatggac 780
   aagtctttta tacagaaatg gaccagcgat atttatgatt acagtaagtc tatcggccgg 840
   gaaggatttt ttttcttcgg tgaatggttt ggtgccagtg cgaatactac aacaggtgtt 900
   gatggtaatg ctatcgatta cgccaacact tccgggtcag cgttgctgga ttttggattc 960
   cgcgatactt tagaaagagt tttggtagga cgtagcggaa atacaatgaa aacgttaaat 1020
   agttatctga taaaaagaca aacagtcttt accagtgatg actggcaggt tgtttttatg 1080
   gataaccatg atatggcacg cattggtacc gctctgcgtt caaacgccac tacttttggt 1140
   cctggaaata atgaaaccgg tggaagtcag agtgaagctt ttgctcagaa acgtatagac 1200
   ctcggtctgg ttgcgacaat gactgtacgt ggtattcctg ccatttatta tggtactgaa 1260
   cattatgccg ctaactttac ctctaacagt tttggtcaag ttggcagtga tccttacaac 1320
   cgagagaaaa tgccaggatt tgatacggaa agtgaggctt tctccattat taaaacactg 1380
   ggtgacctaa ggaaaagtag cccggcaatt caaaatggaa cttatactga actatgggtt 1440
   aatgatgata tattagtatt tgagcggcgt tctgggaacg atattgttat tgttgcactt 1500
   aatcgtggtg aggctaacac aattaatgtt aaaaatatag cggttcctaa tggggtatat 1560
   ccgagtttga ttgggaataa tagtgtttca gtagcaaata aacggacaac actaacactt 1620
   atgcaaaatg aagctgttgt cattcgctca caatcagatg atgcggagaa ccctacagta 1680
   caaagcataa acttcacatg taataacggt tatacgattt caggtcaaag tgtttatatt 1740
   attggtaata tacctcagtt aggtggttgg gacttaacta aagcggtaaa aatatcaccg 1800
   acacaatatc cacaatggag tgcgagctta gagcttcctt ctgacttaaa tgttgaatgg 1860
   aagtgtgtga aacgtaatga aaccaatccg acggctaatg ttgagtggca gtctggtgca 1920
   aataaccagt tcaatagcaa tgacacacaa acaacgaatg gctcgtttta attaaaattt 1980
   agtggaccag cgttccaatc gatggtccac tattcgtact ccggccataa ttatttttga 2040
   ctaatactct tacaaatttt caacc 2065
<210> 5
   <211> 700
   <212> DNA
   <213> Artificial Sequence
<220>
   <223> DNA-Sequenz einer phoA-IFNalpha2b-Genfusion
<400> 5
   attctgaaat gagctgttga caattaatca tcggctcgta taatgtgtgg aattgtgagc 60
   ggataacaat ttcacacagg aaacagaatt ctaaggagga aattatatga aacaaagcac 120
   tattgcactg gcactcttac cgttactgtt tacccctgtg acaaaagctt gtgacttacc 180
   tcagacccat tcactgggct cacgccgtac gctgatgctg ttagcacaga tgcgtcgcat 240
   ttctctgttt agttgtttga aagaccgtca tgattttggg ttcccgcaag aagagtttgg 300
   taatcagttt cagaaagccg aaactattcc ggttctgcac gaaatgattc aacagatttt 360
   taacctgttt tcgacaaagg atagctctgc cgcgtgggat gaaaccttac tggataagtt 420
   ctacaccgaa ctgtaccagc aactgaatga tctggaagca tgcgttatcc agggcgtggg 480
   tgtcacagaa actccgctga tgaaggagga cagcattctg gcggtgcgca aatatttcca 540
   gcgtatcacg ctgtatctga aagagaaaaa atattcgcca tgcgcgtggg aggtcgtgcg 600
   cgcggagatc atgcgcagtt tctctttgag caccaacctg caagaatcct tgcgttccaa 660
   agaataatag tctagaagct tggctgtttt ggcggatgag                       700
<210> 6
   <211> 700
   <212> DNA
   <213> Artificial Sequence
<220>
   <223> DNA-Sequenz einer ompA-IFNalpha2b-Genfusion
<400> 6
   attctgaaat gagctgttga caattaatca tcggctcgta taatgtgtgg aattgtgagc 60
   ggataacaat ttcacacagg aaacagaatt ctaaggagga aattatatga aaaagacagc 120
   tatcgcgatt gcagtggcac tggctggttt cgctaccgta gcgcaggctt gtgacttacc 180
   tcagacccat tcactgggct cacgccgtac gctgatgctg ttagcacaga tgcgtcgcat 240
   ttctctgttt agttgtttga aagaccgtca tgattttggg ttcccgcaag aagagtttgg 300
   taatcagttt cagaaagccg aaactattcc ggttctgcac gaaatgattc aacagatttt 360
   taacctgttt tcgacaaagg atagctctgc cgcgtgggat gaaaccttac tggataagtt 420
   ctacaccgaa ctgtaccagc aactgaatga tctggaagca tgcgttatcc agggcgtggg 480
   tgtcacagaa actccgctga tgaaggagga cagcattctg gcggtgcgca aatatttcca 540
   gcgtatcacg ctgtatctga aagagaaaaa atattcgcca tgcgcgtggg aggtcgtgcg 600
   cgcggagatc atgcgcagtt tctctttgag caccaacctg caagaatcct tgcgttccaa 660
   agaataatag tctagaagct tggctgtttt ggcggatgag 700
<210> 7
   <211> 727
   <212> DNA
   <213> Artificial Sequence
<220>
   <223> DNA-Sequenz einer cgt-IFNalpha2b-Genfusion
<400> 7
   attctgaaat gagctgttga caattaatca tcggctcgta taatgtgtgg aattgtgagc 60
   ggataacaat ttcacacagg aaacagaatt ctaaggagga aattatatga aaagaaaccg 120
   tttttttaat acctcggctg ctattgccat ttcgattgca ttaaatactt ttttttgtag 180
   catgcagacg attgcttgtg acttacctca gacccattca ctgggctcac gccgtacgct 240
   gatgctgtta gcacagatgc gtcgcatttc tctgtttagt tgtttgaaag accgtcatga 300
   ttttgggttc ccgcaagaag agtttggtaa tcagtttcag aaagccgaaa ctattccggt 360
   tctgcacgaa atgattcaac agatttttaa cctgttttcg acaaaggata gctctgccgc 420
   gtgggatgaa accttactgg ataagttcta caccgaactg taccagcaac tgaatgatct 480
   ggaagcatgc gttatccagg gcgtgggtgt cacagaaact ccgctgatga aggaggacag 540
   cattctggcg gtgcgcaaat atttccagcg tatcacgctg tatctgaaag agaaaaaata 600
   ttcgccatgc gcgtgggagg tcgtgcgcgc ggagatcatg cgcagtttct ctttgagcac 660
   caacctgcaa gaatccttgc gttccaaaga ataatagtct agaagcttgg ctgttttggc 720
   ggatgag 727
<210> 8
   <211> 727
   <212> DNA
   <213> Artificial Sequence
<220>
   <223> DNA-Sequenz einer AFA-IFNalpha2b-Genfusion
<400> 8
   attctgaaat gagctgttga caattaatca tcggctcgta taatgtgtgg aattgtgagc 60
   ggataacaat ttcacacagg aaacagaatt ctaaggagga aattatatga aaagaaaccg 120
   tttttttaat acctcggctg ctattgccat ttcgattgca ttacagatct tttttccgtc 180
   cgcttccgct ttcgcttgtg acttacctca gacccattca ctgggctcac gccgtacgct 240
   gatgctgtta gcacagatgc gtcgcatttc tctgtttagt tgtttgaaag accgtcatga 300
   ttttgggttc ccgcaagaag agtttggtaa tcagtttcag aaagccgaaa ctattccggt 360
   tctgcacgaa atgattcaac agatttttaa cctgttttcg acaaaggata gctctgccgc 420
   gtgggatgaa accttactgg ataagttcta caccgaactg taccagcaac tgaatgatct 480
   ggaagcatgc gttatccagg gcgtgggtgt cacagaaact ccgctgatga aggaggacag 540
   cattctggcg gtgcgcaaat atttccagcg tatcacgctg tatctgaaag agaaaaaata 600
   ttcgccatgc gcgtgggagg tcgtgcgcgc ggagatcatg cgcagtttct ctttgagcac 660
   caacctgcaa gaatccttgc gttccaaaga ataatagtct agaagcttgg ctgttttggc 720
   ggatgag 727
<210> 9
   <211> 21
   <212> PRT
   <213> Escherichia coli
<220>
   <223> Aminosaeure-Sequenz des phoA-Signalpeptids von Escherichia coli
<400> 9
   Met Lys Gln Ser Thr Ile Ala Leu Ala Leu Leu Pro Leu Leu Phe Thr
   1 5 10 15
   Pro Val Thr Lys Ala
   20
<210> 10
   <211> 21
   <212> PRT
   <213> Escherichia coli
<220>
   <223> Aminosaeure-Sequenz des ompA-Signalpeptids von Escherichia coli
<400> 10
   Met Lys Lys Thr Ala Ile Ala Ile Ala Val Ala Leu Ala Gly Phe Ala
   1 5 10 15
   Thr Val Ala Gln Ala
   20
<210> 11
   <211> 329
   <212> DNA
   <213> Artificial Sequence
<220>
   <223> artefizielles DNA-Fragment, welches die Sequenz des AFA-Signalpeptids enthaelt
<400> 11
   attctgaaat gagctgttga caattaatca tcggctcgta taatgtgtgg aattgtgagc 60
   ggataacaat ttcacacagg aaacagataa tgaaaagaaa ccgttttttt aatacctcgg 120
   ctgctattgc catttcgatt gcattacaga tcttttttcc gtccgcttcc gctttcgctg 180
   ctgaaccaga agaaacttat cttgattttc gtaaggagac gatatatttt ctattccttg 240
   atcgtttcag cgatggagat ccaagtaata atgcagggtt taattctgca acctacgatc 300
   ctaataattt aaaaaaatat actggagga 329
<210> 12
   <211> 769
   <212> DNA
   <213> Artificial Sequence
<220>
   <223> DNS-Molekuel, welches das Gen der schweren Kette des Anti-Lysozym-Fab-Fragments enthaelt
<220>
   <221> sig_peptide
   <222> (24)..(86)
   <223> ompA-Signalpeptid
<220>
   <221> allele
   <222> (87)..(758)
   <223> Gen der schweren Kette des Anti-Lysozym-Fab-Fragments inkl. HisTag (bp 738-755)
<400> 12
   cagaattcta aggaggaaat tatatgaaaa agacagctat cgcgattgca gtggcactgg 60
   ctggtttcgc taccgtagcg caggctgaag ttaaactgca agaatccggt ccgggtctgg 120
   ttgctccgtc ccagtccctg tccatcacct gcaccgtttc cggtttctcc ctgaccggtt 180
   acggtgttaa ctgggttcgt cagccgccgg gtaaaggtct ggaatggctg ggtatgatct 240
   ggggtgacgg taacaccgac tacaactccg ctctgaaatc ccgtctgtcc atctccaaag 300
   acaactccaa atcccaggtt ttcctgaaaa tgaactccct gcacaccgac gacaccgctc 360
   gttactactg cgctcgtgaa cgtgactacc gtctggacta ctggggtcag ggtaccaccg 420
   ttaccgtttc ctccgctaaa accaccccgc cgtccgttta cccgctggct ccgggttccg 480
   ctgctcagac caactctatg gttaccctgg gttgcctggt taaaggttac ttcccggaac 540
   cggttaccgt tacctggaac tccggttccc tgtcctccgg ttgccacacc ttcccggctg 600
   ttctgcaatc cgacctgtac accctgtcct cctccgttac cgttccgtcc tccacctggc 660
   cgtccgaaac cgttacctgc aacgttgctc acccggcttc ctccaccaaa gttgacaaaa 720
   aaatcgttcc gcgtgaccat caccaccatc accattaata actgcagaa            769
<210> 13
   <211> 768
   <212> DNA
   <213> Artificial Sequence
<220>
   <223> DNS-Molekuel, welches das Gen der leichten Kette des Anti-Lysozym-Fab-Fragemnets enthaelt
<220>
   <221> sig_peptide
   <222> (26)..(115)
   <223> CGTase-Signalsequenz
<220>
   <221> allele
   <222> (116)..(757)
   <223> Gen der leichten Kette des Anti-Lysozym-Fab-Fragments
<400> 13
   aactgcagta catggagaaa ataaaatgaa aagaaaccgt ttttttaata cctcggctgc 60
   tattgccatt tcgattgcat taaatacttt tttttgtagc atgcagacga ttgctgacat 120
   cgaactgacc cagtccccgg cttccctgtc cgcttccgtt ggtgaaaccg ttaccatcac 180
   ctgccgtgct tccggtaaca tccacaacta cctggcttgg taccagcaga aacagggtaa 240
   atccccgcag ctgctggttt actacaccac caccctggct gacggtgttc cgtcccgttt 300
   ctccggttcc ggttccggta cccagtactc cctgaaaatc aactccctgc aaccggaaga 360
   cttcggttcc tactactgcc agcacttctg gtccaccccg cgtaccttcg gtggtggtac 420
   caaactggaa ctgaaacgtg ctgacgctgc tccgaccgtt tccatcttcc cgccgtcctc 480
   cgaacagctg acctccggtg gtgcttccgt tgtttgcttc ctgaacaact tctacccgaa 540
   agacatcaac gttaaatgga aaatcgacgg ttccgaacgt cagaacggtg ttctgaactc 600
   ctggaccgac caggactcca aagactccac ctactccatg tcctccaccc tgaccctgac 660
   caaagacgaa tacgaacgtc acaactccta cacctgcgaa gctacccaca aaacctccac 720
   ctccccgatc gttaaatcct tcaaccgtaa cgaataatag ctgcagaa              768

## Beschreibung

Die Erfindung betrifft ein Signalpeptid zur Produktion von rekombinanten Proteinen.

Die großvolumige wirtschaftliche Herstellung von rekombinanten Proteinen ist für die biotechnologische und pharmazeutische Industrie von wachsender Bedeutung. Generell werden rekombinante Proteine entweder in Säugerzellkulturen oder in mikrobiellen Systemen hergestellt. Mikrobielle Systeme weisen gegenüber Säugerzellkulturen den Vorteil auf, dass auf diese Weise rekombinante Proteine in kürzerer Zeit und zu geringeren Kosten hergestellt werden können. Für die Produktion von rekombinanten Proteinen eignen sich daher vor allem Bakterien, bevorzugt solche der Gattung Escherichia, besonders bevorzugt E. coli. In E. coli können rekombinante Proteine prinzipiell auf verschiedene Arten hergestellt werden:
1. intrazelluläre Produktion als lösliches Protein;
2. intrazelluläre Produktion als Einschlusskörperchen ("inclusion bodies");
3. Sekretion in das Periplasma oder in das Nährmedium.

Der Produktionsprozess des rekombinanten Proteins gliedert sich dabei immer in zwei Teile. Der erste Teil ist die Fermentation, welche zum Rohprodukt führt. Als Rohprodukt wird in diesem Fall das Fermentationsergebnis bezeichnet, welches das rekombinante Protein und außerdem noch verunreinigende wirtseigene Proteine enthält. Der zweite Teil des Produktionsprozesses umfasst die Aufreinigung des rekombinanten Proteins ausgehend vom Rohprodukt.

Aufwand und Kosten der Herstellung des rekombinanten Proteins werden neben den Produktionskosten des Rohproduktes, welches direkt nach der Fermentation als Gemisch umfassend das rekombinante Proteine sowie Wirtsproteine vorliegt, auch wesentlich bestimmt durch die Aufreinigungskosten des Rohproduktes zum erwünschten rekombinanten Protein. Die Aufreinigung erfolgt in den meisten Fällen über mehrere Stufen mittels chromatographischer Verfahren. Dabei spielt die Abreinigung von verunreinigenden Wirtsproteinen, die z. T. immunogen oder toxisch sind, eine bedeutende Rolle.

Die Sekretion von Proteinen in E. coli erfolgt in den meisten Fällen über den sogenannten sec-pathway (Driessen et al., 1998). Dieses System ist für den Export von bestimmten bakterieneigenen Proteinen verantwortlich. Die Gene für diese Proteine weisen am 5'-Ende jeweils eine sogenannte Signalsequenz auf. Bei der Proteinsynthese wird dieses in ein Signalpeptid übersetzt und bewirkt die Sekretion des Proteins durch die Cytoplasma-Membran. Nach der Sekretion wird das Signalpeptid durch das Enzym Signalpeptidase abgespalten und das reife Protein freigesetzt.

Das sec-System kann auch zur Sekretion von rekombinanten, wie z. B. heterologen Proteinen verwendet werden (Lee et al., Methods in Molecular Biology 308, 2005). Dafür wird das rekombinante Gen für das zu produzierende rekombinante Protein mit einer Signalsequenz verknüpft ("in frame Fusion"), was zur Produktion einer Signalpeptid-Protein-Fusion führt. Das durch die Signalsequenz codierte Signalpeptid vermittelt die Sekretion des rekombinanten Proteins über die Cytoplasma-Membran in das Periplasma durch das bakterieneigene sec-System. Dabei wird das Signalpeptid an der Schnittstelle zwischen Signalpeptid und dem rekombinanten Protein abgespalten und das gewünschte rekombinante Protein im Periplasma erhalten. Das rekombinante Protein kann danach aus dem Periplasma gereinigt werden.

Die Sekretion bietet im Vergleich zu den anderen Herstellungsverfahren den Vorteil, dass das rekombinante Protein direkt als natives, lösliches, korrekt gefaltetes Protein erhalten wird, welches im Vergleich zum "inclusion body"-Verfahren nicht denaturiert und wieder renaturiert werden muss; ein Schritt, der mit hohen Verlusten an Ausbeute einhergeht. Außerdem ist das Rohprodukt in diesem Fall im Vergleich zur intrazellulären, löslichen Produktion mit weniger Wirtsproteinen verunreinigt, da das Periplasma von Bakterien weitaus weniger Wirtsproteine enthält, als das Cytoplasma.

Unter bestimmten Bedingungen oder in bestimmten BakterienStämmen wird das rekombiante Protein aus dem Periplasma in das Nährmedium freigesetzt (z. B. Ray et al., 2002; EP0338410B1; Nagahari et al., 1985; Yang et al., 1998; EP0677109B1) und kann aus diesem aufgereinigt werden.

Die Sekretion der Proteine in das Nährmedium bietet, verglichen mit der Sekretion ins Periplasma, den weiteren Vorteil, dass das Protein dann in noch reinerer Form vorliegt. Außerdem ist als erster Aufreinigungsschritt keine aufwändige Präparation des Periplasmas oder ein Aufschluss der Zelle nötig, sondern eine viel einfachere und reproduzierbar verlaufende Abtrennung der ganzen Zellen.

Wie erwähnt, wird für die Sekretion eines zu produzierenden Proteins das dafür codierende Gen mit einer Signalsequenz verknüpft, welche bewirkt, dass das zu produzierende Protein zunächst als Fusion mit dem von der Signalsequenz codierten Signalpeptid produziert wird. Dieses Signalpeptid bewirkt die Sekretion des produzierten Proteins.

Signalpeptide sind aus drei Regionen aufgebaut: die N-terminale N-Region (1-5 Aminosäuren) enthält in der Regel ein oder mehrere Aminosäuren, die positive Ladung tragen. Die in der Mitte liegende H-Region besteht meist aus 7 - 15 Aminosäuren, von denen viele hydrophob sind. Die in der Regel 3 - 7 Aminosäuren umfassende C-Region enthält an Position -1 und -3 vor der Schnittstelle meist neutrale, kurzkettige Aminosäuren (A, G, S, T, C).

Verschiedene Signalsequenzen und die zugehörigen Signalpeptide sind im Stand der Technik beschrieben, z. B. phoA, ompA, pelB, ompF, ompT, lamB, malE, Staphylococcal protein A, stII (Choi & Lee, 2004; EP0396612B1).

Das Signalpeptid der Cyclodextrin-Glycosyl-Transferase (CGTase) aus verschiedenen Stämmen, wie z. B. Klebsiella oxytoca (Klebsiella pneumoniae M5a1), und seine Verwendung zur Sekretion von CGTase in E. coli-Stämmen ist in US5395927 beschrieben.
Ebenfalls ist beschrieben (EP0448093B1), dass ein rekombinantes Protein, wie zum Beispiel ein Hirudin-Derivat, in E. coli-Stämmen produziert und sekretiert werden kann, indem das Gen für das rekombinante Protein mit der Signalsequenz der CGTase fusioniert wird. Im Falle eines speziellen Hirudin-Derivates führt dies zu einer Ausbeute von 250 mg/l in einer Schüttelkolben-Anzucht und zu 2,63 g/l in einer Fermentation. EP0448093B1 beschreibt allerdings auch, dass bei einem anderen rekombinanten Protein nur Ausbeuten von bis zu 25 mg/l erhalten wurden. Das Signalpeptid der CGTase ist - wie alle anderen bekannten Signalpeptide auch - also nicht in der Lage, die Sekretion eines beliebigen rekombinanten Proteins in gleich hohen Ausbeuten zu vermitteln. Da jedes rekombinante Protein von einer eigenen DNS Sequenz codiert wird und insbesondere die DNS-Sequenz an der Übergangsstelle zwischen Signalsequenz und der für das rekombinante Protein codierenden Sequenz damit unterschiedlich ist, muss in der Regel für jedes rekombinante Protein ein optimales Signalpeptid gefunden werden.

WO01/94418A offenbart synthetische Signalpeptide, welche am C-Terminus die Sequenz Z-x-Z besitzen, wobei Z vorzugsweise Alanin ist.

WO01/21662A offenbart ein Signalpeptid einer CGTase und dessen Verwendung zur Produktion und Sekretion von Ala-Hirudin in E.coli.

Eine Aufgabe der vorliegenden Erfindung ist es, neue Signalpeptide bereitzustellen.

Die Aufgabe wird gelöst durch ein Signalpeptid, das dadurch gekennzeichnet ist, dass es eine Aminosäuresequenz:
MKRNRFFNTSAAIAISIALQIFFPSASAFA (SEQ ID NO 1) oder eine Aminosäuresequenz, bei der im Vergleich zu SEQ ID NO 1 eine bis zehn bevorzugt eine bis fünf und insbesondere bevorzugt eine bis drei Aminosäuren, mit Ausnahme der drei letzten Aminosäuren vor der Schnittstelle, verändert sind, hat und seine drei letzten Aminosäuren vor der Schnittstelle Alanin-Phenylalanin-Alanin (AFA) sind.

Wie beschrieben, besteht ein Signalpeptid aus verschiedenen Bereichen, die Aminosäuren einer bestimmten Gruppe (z. B. geladen oder hydrophob oder kurzkettig) enthalten. Der Fachmann kann daher durch einen Ersatz einer Aminosäure durch eine andere mit vergleichbaren Eigenschaften ein neues Signalpeptid mit unveränderten Eigenschaften erzeugen.

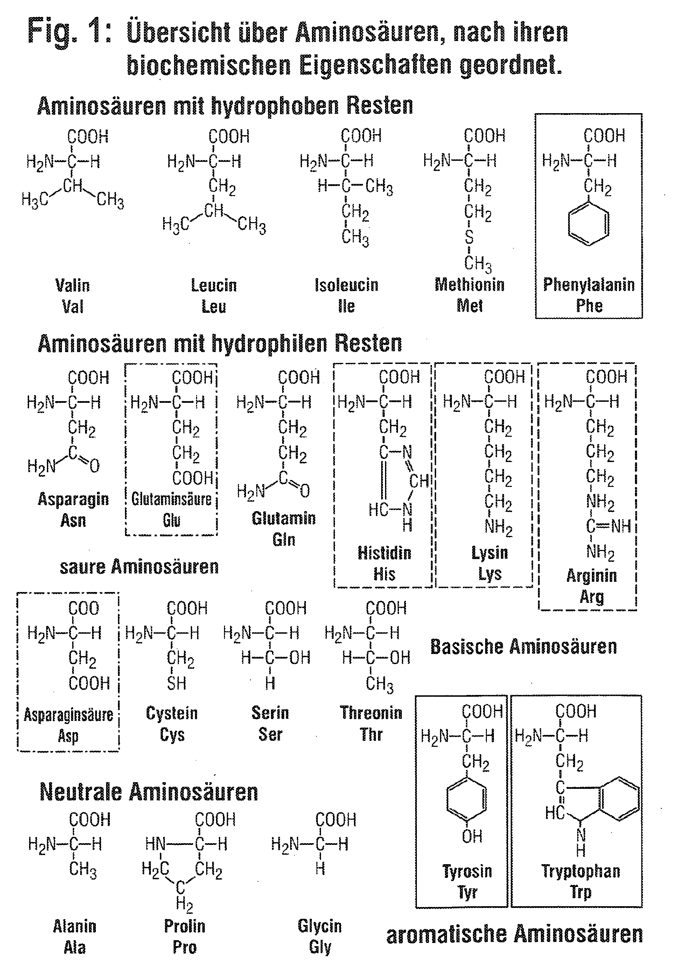

Aus diesem Grund sind als erfindungsgemäße Signalpeptide auch solche anzusehen, in denen im Vergleich zu SEQ ID NO 2 1 - 10, besonders bevorzugt 1 - 5, insbesondere bevorzugt 1 - 3 Aminosäuren mit Ausnahme der letzten drei Aminosäuren vor der Schnittstelle, verändert sind. Vorzugsweise handelt es sich dabei um Austausche von Aminosäuren, die ähnliche biochemische Eigenschaften haben, wie z. B. basische Aminosäuren (Lysin, Argining, Histidin) gegen basische, saure Aminosäuren (Aspartat, Glutamat, Asparagin, Glutamin) gegen saure, hydrophobe gegen hydrophobe etc. Eine Übersicht über die biochemischen Eigenschaften von Aminosäuren zeigt Fig 1.

Die Erfindung betrifft ferner die für das erfindungsgemäße Signalpeptid kodierende Signalsequenz.

Es handelt sich dabei um eine Signalsequenz mit der DNS-Sequenz
ATGAAAAGAAACCGTTTTTTTAATACCTCGGCTGCTATTGCCATTTCGATTGCATTACAGAT CTTTTTTCCGTCCGCTTCCGCTTTCGCT (SEQ ID NO 2) sowie alle DNS-Sequenzen, die aufgrund des degenerierten genetischen Codes für die Aminosäuresequenz SEQ ID NO 1 codieren.

Im Rahmen eines Screenings wurden verschiedene Signalsequenzen, die durch Veränderung der CGTase Signalsequenz (MKRNRFFNTSAAIAISIALNTFFCSMQTIA, SEQ ID NO 3) erhalten wurden, hinsichtlich ihrer Eigenschaften verglichen. Überraschend wurde gefunden, dass die erfindungsgemäße Signalsequenz sich dafür eignet, ein größeres Spektrum an rekombinanten Proteinen in höherer Ausbeute in Wirtszellen zu produzieren und sekretieren, als die CGTase Signalsequenz oder auch andere Signalsequenzen.

Eine erfindungsgemäße DNS-Sequenz kann durch Gensynthese oder Ligation von entsprechenden Oligonukleotiden nach dem Fachmann bekannten Methoden erhalten werden. Die erfindungsgemäße DNS-Sequenz wird in frame nach dem Fachmann bekannten Verfahren (z. B. analog Lee et al., 2005) mit dem Gen des zu produzierenden rekombinanten Proteins verknüpft und kann in einen Vektor eingebracht werden.

Vorzugsweise wird diese Kombination von Signalsequenz und rekombinantem Gen mit in E. coli funktionellen Expressionssignalen versehen (Promotor, Transkriptions-, Translationsstart, Ribosomenbindestelle). Als Promotoren eignen sich alle dem Fachmann bekannten Promotoren, wie einerseits beispielsweise induzierbare Promotoren, wie der lac-, tac-, trc-, lambda PL, ara- oder tet-Promotor oder davon abgeleitete Sequenzen. Andererseits kann auch eine konstitutive Expression erfolgen durch Verwendung eines konstitutiven Promotors, wie z. B. des GAPDH-Promotors. Es kann aber auch der normalerweise mit dem Gen des zu produzierenden rekombinanten Proteins verknüpfte Promotor Verwendung finden.

Die Erfindung betrifft somit auch ein Expressionskonstrukt umfassend ein Expressionssignal, eine erfindungsgemäße Signalsequenz sowie ein in frame verknüpftes rekombinantes Gen kodierend für ein rekombinantes Protein, das produziert werden soll.

Dieses erfindungsgemäße Expressionskonstrukt wird unter Verwendung von dem Fachmann bekannter Methoden in eine Wirtszelle eingebracht. Dies erfolgt z. B. auf einem Vektor, z. B. einem Plasmid, wie etwa einem Abkömmling eines bekannten Expressionsvektors, wie pUC18, pBR322, pACYC184, pASK-IBA3 oder pET. Als Selektionsmarker für Plasmide geeignet sind Gene, die für eine Resistenz gegenüber z. B. Ampicillin, Tetracyclin, Chloramphenicol, Kanamycin oder andere Antibiotika codieren.

Daher sind auch Plasmide, die die erfindungsgemäße Signalsequenz oder ein erfindungsgemäßes Expressionskonstrukt enthalten, Gegenstand der Erfindung.

Bei dem rekombinanten Protein handelt es sich vorzugsweise um ein heterologes Protein. Bevorzugt handelt es sich bei dem rekombinanten Protein um ein Protein, das Verwendung in technischen Ansätzen findet oder um ein Protein, das als Pharmawirkstoff eingesetzt wird (Biologics, Biopharmazeutikum). Solche Proteine sind beispielsweise Hirudin, Insulin, Interferone, wie alpha- oder beta-Interferon (z. B. Interferonα2b), Antikörper oder Antikörper-Fragement (wie z. B. Fab-Fragmente, scFv) oder andere Bindeproteine oder Enzyme, wie CGTase.

Das erfindungsgemäße Expressionskonstrukt wird nach dem Fachmann bekannten Methoden in eine Mikroorganismenzelle (Wirtszelle) eingebracht. In der Folge kann das erfindungsgemäße Expressionskonstrukt weiterhin als Plasmid in der Wirtszelle vorliegen oder in das Chromosom der Wirtszelle integriert werden.

Daher sind auch gentechnisch veränderte Mikroorganismenzellen, die ein erfindungsgemäßes Expressionskonstrukt oder ein erfindungsgemäßes Plasmid enthalten, Gegenstand der Erfindung.

Bei den Wirtszellen, handelt es sich um Zellen eines Bakterienstammes aus der Familie der Enterobacteriaceae, bevorzugt um einen Stamm der Art Escherichia coli. Besonders bevorzugt ist ein Escherichia (E.) coli-Stamm, der sich dadurch auszeichnet, dass er nach Transformation mit dem erfindungsgemäßen Expressionskonstrukt nach Fermentation eine höhere Konzentration des rekombinanten Proteins im Periplasma oder im Nährmedium aufweist als der Stamm E. coli W3110 (ATCC 27325) nach Transformation mit dem erfindungsgemäßen Expressionskonstrukt.

Ganz besonders bevorzugt handelt es sich um einen der folgenden E. coli-Stämme:
- BLR: Ray et al., 2002, käuflich erhältlich bei Novagen
- K802 = CGSC***** 5610: Yang et al., 1998
- WCM105: herstellbar gemäß EP0338410B1
- MM28 = CGSC***** #5892: Nagahari et al., 1985
- RV308 = ATCC****** 31608; EP0677109B1
- RR1 : ATCC****** 31434: Nagahari et al., 1985
   * käuflich erhältlich über das E. coli Genetic Stock Center CGSC (830 Kline Biology Tower, MCD Biology Department, 266 Whitney Ave., PO box 208103, Yale University, New Haven,
   ** käuflich erhältlich über LGC Promochem, Mercatorstr. 51, 46485 Wesel, Deutschland.

Die Sekretion des produzierten Proteins erfolgt durch den sec-Apparat der Wirtszelle. Nach erfolgter Sekretion in das Periplasma wird von einer Signalpeptidase (z. B. LepB bei E. coli) das erfindungsgemäße Signalpeptid abgespalten und es entsteht das gewünschte rekombinante Protein.

Die Erfindung betrifft somit auch ein Verfahren zur fermentativen Herstellung eines rekombinanten Proteins mittels einer Wirtszelle enthaltend das erfindungsgemäße Expressionskonstrukt in einem Fermentationsmedium. Dieses Verfahren ist dadurch gekennzeichnet, dass ein erfindungsgemäßer Wirtsstamm in einem Fermentationsmedium kultiviert wird, der Wirtsstamm das rekombinante Protein in Form einer in frame Signalpeptid-Protein-Fusion produziert, wobei das Signalpeptid ein erfindungsgemäßes Signalpeptid ist und das Signalpeptid bei der Sekretion der Signalpeptid-Protein-Fusion über die Cytoplasma-Membran in das Periplasma an der Schnittstelle zwischen Signalpeptid und dem rekombinanten Protein abgespalten wird und das gewünschte rekombinante Protein im Periplasma oder dem Fermentationsmedium erhalten wird und das rekombinante Protein nach der Fermentation aufgereinigt wird.

Das rekombinante Protein wird in einer Fermentation in das Periplasma oder vorzugsweise in das Fermentationsmedium sekretiert.

Das rekombinante Protein kann entweder aus dem Periplasma der Wirtszellen oder vorzugsweise aus dem Fermentationsmedium nach dem Abtrennen der Zellen aufgereinigt werden.

Die Fermentation des Bakterienstammes zur erfindungsgemäßen Produktion des rekombinanten Proteins erfolgt vorzugsweise in einem Vollmedium oder Minimalsalzmedium. Diese Medien sind aus der Literatur bekannt.

Als Kohlenstoffquelle können prinzipiell alle verwertbaren Zucker, Zuckeralkohole, organische Säuren bzw. deren Salze, Stärkehydrolysate, Melasse oder andere Stoffe verwendet werden. Dabei werden bevorzugt Glucose oder Glycerin eingesetzt. Auch ist eine kombinierte Fütterung mehrerer verschiedener Kohlenstoffquellen möglich. Als Stickstoffquellen können Harnstoff, Ammoniak und seine Salze, Nitratsalze sowie andere N-Quellen dienen. Zu den möglichen Stickstoffquellen gehören auch komplexe Aminosäuregemische, wie Hefeextrakt, Pepton, Malzextrakt, Sojapepton, Casaminosäuren, Maisquellwasser (Corn Steep Liquor) und NZ-Amine (z. B. Kerry Bio-Science, Chicago, USA).

Außerdem können dem Medium weitere Komponenten zugegeben werden wie Vitamine, Salze, Hefeextrakt, Aminosäuren und Spurenelemente, durch die das Zellwachstum verbessert wird.

Die Inkubation des Stammes erfolgt vorzugsweise unter aeroben Kultivierungsbedingungen über einen Zeitraum von 16 - 150 h und im Bereich der für den jeweiligen Stamm optimalen Wachstumstemperatur.

Als optimaler Temperaturbereich werden 15 - 55°C bevorzugt. Besonders bevorzugt ist eine Temperatur zwischen 28 und 37°C.

Die Anzucht des Stammes kann im Schüttelkolben oder Fermenter erfolgen, wobei keine Beschränkungen hinsichtlich Volumen gegeben sind. Die Anzucht kann im Batch-Verfahren, im Fed-Batch- oder in einem kontinuierlichen Verfahren erfolgen.

Die Expression des rekombinanten Proteins erfolgt dabei entweder konstitutiv, d.h. nicht-induziert, oder durch Induktion mittels physikalischer oder physiologischer Stimuli. Induziert werden kann die Expression zum Beispiel durch Zusatz einer den Promotor induzierenden Substanz, wie etwa Lactose oder IPTG beim lac- oder tac-Promotor.

Die Aufreinigung von Proteinen aus dem Periplasma bzw. dem Kulturmedium kann nach dem Fachmann bekannten Verfahren, wie Aufbruch oder Abtrennung der Zellen, chromatographische Reinigung, Komplexierung, Filtration oder Fällung des Proteins erfolgen.
Fig. 1 zeigt eine Übersicht von Aminosäuren.
Fig. 2 zeigt die Plasmidkarte von Plasmid pJF118ut.
Fig. 3 zeigt die Plasmidkarte von Plasmid pCGT.
Fig. 4 zeigt die Plasmidkarte von Plasmid pKP651.
Fig. 5 zeigt die Plasmidkarte von Plasmid pKP652.
Fig. 6 zeigt die Plasmidkarte von Plasmid pKPIFN.
Fig. 7 zeigt die Plasmidkarte von Plasmid pBaBIFN1.
Fig. 8 zeigt die Plasmidkarte von Plasmid pFab-Anti-Lysozym.
Fig. 9 zeigt einen Immunoblot, in dem Kulturüberstände von Interferonα2b produzierenden Zellen (s. Beispiel 2) analysiert werden.

Spur 1: Größenstandard;
Spuren 2 und 3: 20 und 40 ng Interferonα2b;
Spuren 4 - 6: Überstand von Anzuchten mit Plasmid pBaBIFN1 nach 24, 48, 72 h;
Spuren 7 - 9: Überstand von Anzuchten mit Plasmid pKPIFN nach 24, 48, 72 h;
Spuren 10 - 12: Überstand von Anzuchten mit Plasmid pKP651 nach 24, 48, 72 h;
Spuren 13 - 15: Überstand von Anzuchten mit Plasmid pKP652 nach 24, 48, 72 h.
Bei 24 h wurden jeweils 5 µl, bei 48 h und 72 h 1 µl aufgetragen.
Die Zellen enthalten Interferonα2b-Expressionsplasmide, die sich in den Signalsequenzen unterscheiden. Das gebildete Interferonα2b wurde mittels Antikörpern detektiert (Pfeil). Die Verwendung der erfindungsgemäßen Signalsequenz führt zu gesteigerter Interferonα2b-Produktion.

Die folgenden Beispiele dienen der weiteren Erläuterung der Erfindung.

### Beispiel 1: Erzeugung einer erfindungsgemäßen Signalsequenz und eines erfindungsgemäßen Vektors

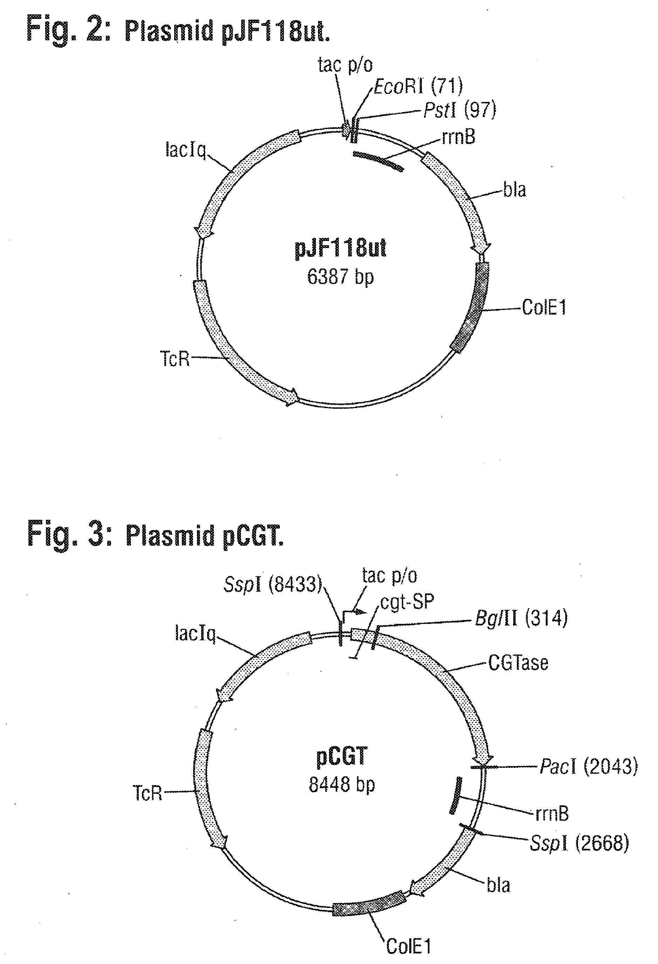

Als Ausgangsplasmid wird das Plasmid pCGT folgendermaßen erzeugt:
Ein DNS-Fragment mit der SEQ ID NO 4, welches ein Cyclodextrin-Glycosyl-Transferase (CGTase)-Gen aus Klebsiella pneumoniae M5a1 enthält (Genbanknr. M15264), wurde per Gensynthese hergestellt. Dieses DNS-Fragment wurde in den Expressionsvektor pJF118ut (Fig. 2) kloniert, welcher bei der DSMZ-Deutsche Sammlung von Mikroorganismen und Zellkulturen GmbH (Braunschweig) unter der Nummer DSM 18596 hinterlegt ist. pJF118ut ist ein Derivat des bekannten Expressionsvektors pKK223-3 (Amersham Pharmacia Biotech) und enthält neben dem β-Lactamase-Gen und dem Tetracyclin-Resistenzgen noch den tac-Promotor, der durch das LacIq-Genprodukt, dessen Gen ebenfalls auf dem Plasmid vorliegt, reprimiert wird und der durch einen Induktor, wie z. B. D-Lactose oder Isopropyl-β-D-thiogalactopyranosid (IPTG), angeschaltet werden kann.
Das Plasmid pJF118ut wurde mit dem Restriktionsenzym EcoRI vollständig geschnitten und die jeweils an den 5'-Enden des linearen DNS-Fragments überstehenden Basen wurden mit Sl-Nuklease abgespalten. Das auf diese Weise vorbereitete Vektor-DNS-Molekül wurde mit dem CGTase-umfassenden DNS-Fragment (SEQ ID NO 4) unter Verwendung der T4-Ligase ligiert. Der Stamm DH5α wurde mit dem Ligationsansatz nach der CaCl₂-Methode transformiert, wobei mittels Ampicillin (100 mg/l) auf plasmidhaltige Zellen selektiert wurde. Aus Ampicillin-resistenten Transformanden wurde das Plasmid wieder isoliert und durch Restriktionsanalyse überprüft. Das auf diese Weise erzeugte Plasmid, bei dem die Expression des CGTase-Gens unter der Kontrolle des tac-Promotors liegt, wurde mit pCGT bezeichnet (Fig. 3).

Das Gen für eine CGTase fusioniert an die Signalsequenz für die CGTase wurde entfernt: Das 8448 bp große Plasmid wurde dazu mit den Restriktionsenzymen SspI und PacI in einem Partialverdau nach dem Fachmann bekannten Methoden geschnitten. Das 6390 bp große Fragment wurde isoliert und mit Klenow-Enzym behandelt, wodurch die Enden geglättet wurden. Das 2058 bp große Fragment wurde entfernt.
Danach wurden folgende vier DNS-Fragmente durch Gensynthese hergestellt:

Alle DNS-Fragmente enthalten die tac-Promotor-Region und das Gen für Interferonα2b und vier unterschiedliche Signalsequenzen (fett markiert). Diese vier verschiedenen Signalsequenzen codieren für folgende vier verschiedene Signalpeptide, wobei die ersten drei Signalpeptide (SEQ ID NO 9, 10, 3) aus dem Stand der Technik bekannt sind und das vierte Signalpeptid (SEQ ID NO 2) erfindungsgemäß ist:
phoA: MKQSTIALALLPLLFTPVTKA
   SEQ ID NO 9
ompA: MKKTAIAIAVALAGFATVAQA
   SEQ ID NO 10
cgt: MKRNRFFNTSAAIAISIALNTFFCSMQTIA
   SEQ ID NO 3
AFA: MKRNRFFNTSAAIAISIALQIFFPSASAFA
   SEQ ID NO 2

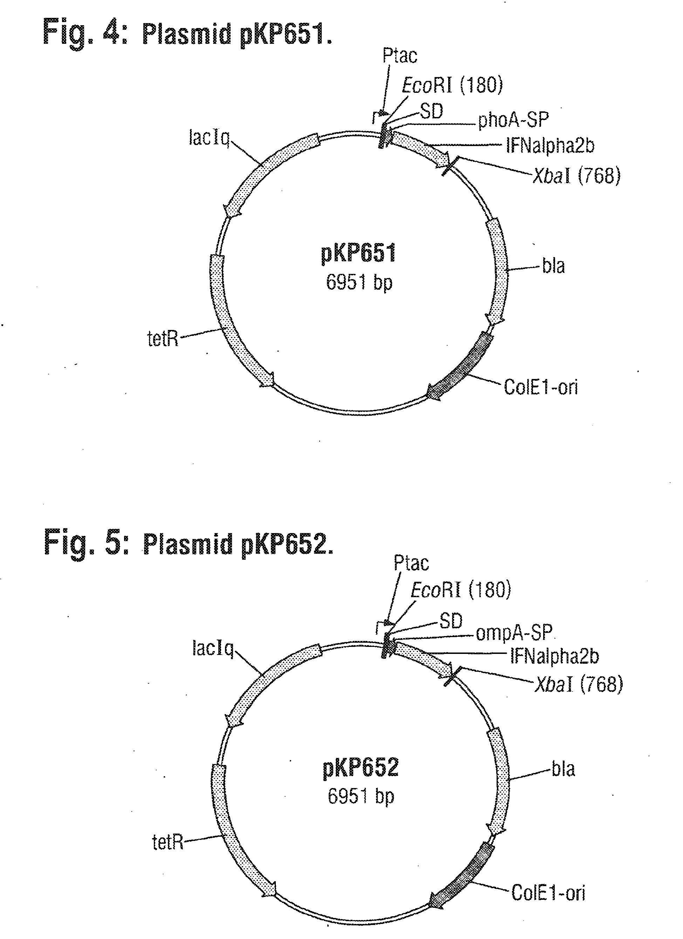

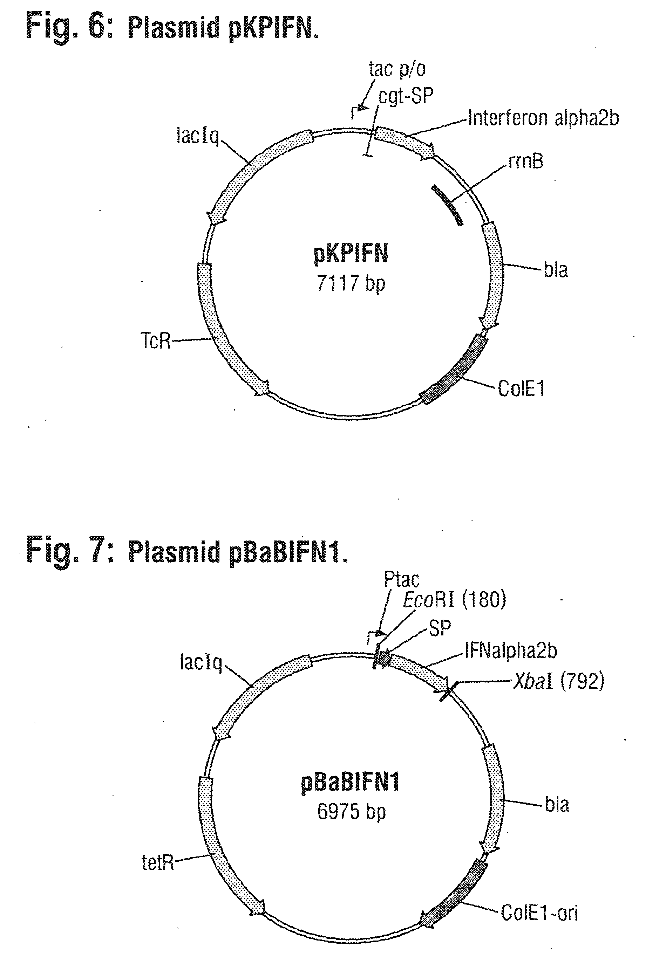

Durch diese Klonierungen entstanden folgende vier Plasmide:
- pKP651 (phoA-Signalsequenz) Fig. 4
- pKP652 (ompA-Signalsequenz) Fig. 5
- pKPIFN (cgt-Signalsequenz) Fig. 6
- pBaBIFN1 (AFA: erfindungsgemäße Signalsequenz) Fig. 7

Diese Plasmide wurden mittels bekannter Methoden in den E. coli-Stamm DH5α eingebracht. Der erfindungsgemäße Stamm DHSα/pBaBIFN1 wurde bei der DSMZ (Deutsche Sammlung für Mikroorganismen und Zellkulturen GmbH, D-38142 Braunschweig) unter der Nummer DSM 18343 gemäß Budapester Vertrag hinterlegt.

### Beispiel 2: Steigerung der Interferon-Produktion durch Verwendung der erfindungsgemäßen Signalsequenz

Plasmid pKP651 (phoA-Signalsequenz), pKP652 (ompA-Signalsequenz), pKPIFN (cgt-Signalsequenz) und pBaBIFN1 (erfindungsgemäße AFA-Signalsequenz)(siehe Beispiel 1) wurden in Stamm WCM105 (herstellbar gemäß EP0338410B1) durch Transformation nach gängiger Methode (mittels CaCl₂-Transformation) eingeführt. Eine Selektion auf plasmidhaltige Stämme erfolgte mittels Ampicillin (100 mg/L).

Folgende Stämme wurden erhalten:
- WCM105/pKP651 (phoA-Signalsequenz)
- WCM105/pKP652 (ompA-Signalsequenz)
- WCM105/pKPIFN (cgt-Signalsequenz)
- WCM105/pBaBIFN1 (AFA-Signalsequenz)

Die Produktion von Interferonα2b in den erhaltenen Stämmen wurde untersucht. Dazu wurden die Stämme in 10 ml LB-Medium mit 100 mg/L Ampicillin und mit 1 % Glucose bei 30°C kultiviert. Bei einer optischen Dichte bei 600 nm (OD600) von 0,5 wird die Produktion von Interferonα2b durch Zugabe von IPTG (Isopropylthiogalactosid) ad 0,5 mM induziert.
Im Kulturüberstand wurde nach 24 h, 48 h und 72 h das gebildete und sekretierte Interferon durch Auftrennung der Proteine im SDS-Gel und Detektion im Immunoblot mit anti-Interferonspezifischen Antikörpern wie folgt quantifiziert:
Je 1 µl (48 und 72 h) bzw 5 µl (24 h) Überstand wurde mit Probenpuffer versetzt (2x Tris SDS - Sample Buffer (Invitrogen Cat.No. LC2676): 0.125 M Tris.HCl, pH 6.8, 4 % w/v SDS, 20 % v/v Glycerin, 0.005 % v/v Bromphenolblau, 5 % beta-Mercaptoethanol). Außerdem wurden definierte Mengen von Interferonα2b als Standard mit aufgetragen. Denaturieren der Proteine erfolgte durch Erhitzen auf 100°C für 5 Min., Abkühlen für 2 Min. auf Eis und abzentrifugieren.
Die Proteine wurden durch Elektrophorese in einem 12 % NuPAGE^{®} Bis-Tris-Gel (Invitrogen Cat.No. NP0341) mit 1x MES-haltigem Running Buffer (Invitrogen Cat.No. NP0002) aufgetrennt (Elektrophorese-Parameter: 40 Min. bei 200 V).
Detektion und Quantifizierung über Immunoblot wurde nach folgender Vorschrift durchgeführt:

### Transfer im Naßblot-Verfahren:

Modul: Amersham: Hoefer TE 22 Mini Tank Transfer Unit, Code Number: 80-6204-26
Membran: Nitrocellulose-Membran (Schleicher&Schuell, BA 85, Cellulosenitrat (E), 0,45 µm Porengröße)
Whatman-Filter und Nitrocellulose-Membran in passende Größe schneiden und mit Schaumstoffstücken (Schwämmen) in Transferpuffer (Invitrogen Cat.No. LC3675) luftblasenfrei tränken. Aufbau des Sandwiches: schwarzes Gitter, Verbindung mit der Kathode, 2 Schwämme, je 3 mm dick, Whatman-Papier, SDS-Polyacrylamidgel, NC-Membran, Whatman, 1 Schwamm, 6 mm dick, weißes Gitter, Verbindung mit der Anode.
Transferbedingungen: I = 200mA constant current, U = unbegrenzt, Laufzeit 60 Min.

### Prehybridisierung

Inkubation der Membran in 25 ml Prehybridisierungspuffer
30 Min. bei RT schwenken

### Hybridisierung 1. Antikörper

Inkubation der Membran in 25 ml Prehybridisierungspuffer + 0,15 µg/ml (-> 3,75 µg) Anti-human-IFN-alpha Antibody (Pepro Tech EC, über Biozol Cat. No.: 500-P32A)
90 Min. oder über Nacht bei RT schwenken

### Waschen

10 Sekunden mit 1 x PBS schwenken, RT, Puffer abgießen
2 x 15 Min. mit 1x PBS schwenken, RT, Puffer abgießen

### Hybridisierung 2. Antikörper

Inkubation der Membran in 25 ml Prehybridisierungspuffer + 25 µl (1:1000) Goat Anti-Rabbit IgG Horseradish Peroxidase Conjugate (HRP) (Southern Biotech, über Biozol Cat. No.: 4050-05) 60 Min. bei RT schwenken

### Waschen

10 Sekunden mit 1 x PBS schwenken, RT, Puffer abgießen
2 x 15 Min. mit 1 x PBS schwenken, RT, Puffer abgießen

### Detektion über Chemilumineszenz

Lumi-Light Western Blotting Substrate (Roche, Cat. No.: 2015200) vorbereiten: Lumi-Light Luminol / Enhancer Solution und Lumi-Light Stable Peroxide Solution im Verhältnis 1:1 mischen: 3 ml / NC-Membran.
Blot 5 Min. bei RT mit Lumi-Light Western Blotting Substrate inkubieren, Überschuss ablaufen lassen, Membran mit Frischhaltefolie bedecken und sofort einen X-Ray Film (Kodak, X-OMAT) auflegen, 2 Min. exponieren, entwickeln und fixieren. Bei schwachen Signalen wird die Exposition über einen längeren Zeitraum wiederholt.

### Puffer

Prehybridisierungspuffer: 5 % Magermilchpulver in 1x PBS
10x PBS: 100 mM NaH₂PO₄, 1,5 M NaCl, pH 7,5 mit NaOH, 0,5 % Triton 100
1x PBS: 10x PBS mit vollentsalztem Wasser 1:10 verdünnen

### Quantifizierung

Eine quantitative Auswertung erfolgte nach Einscannen des Immunoblots mit Biorad GS-800 Calibrated Densitometer mittels der Quantity One 1-D-Analysis Software (Biorad) durch Vergleich mit dem aufgetragenen Standard.

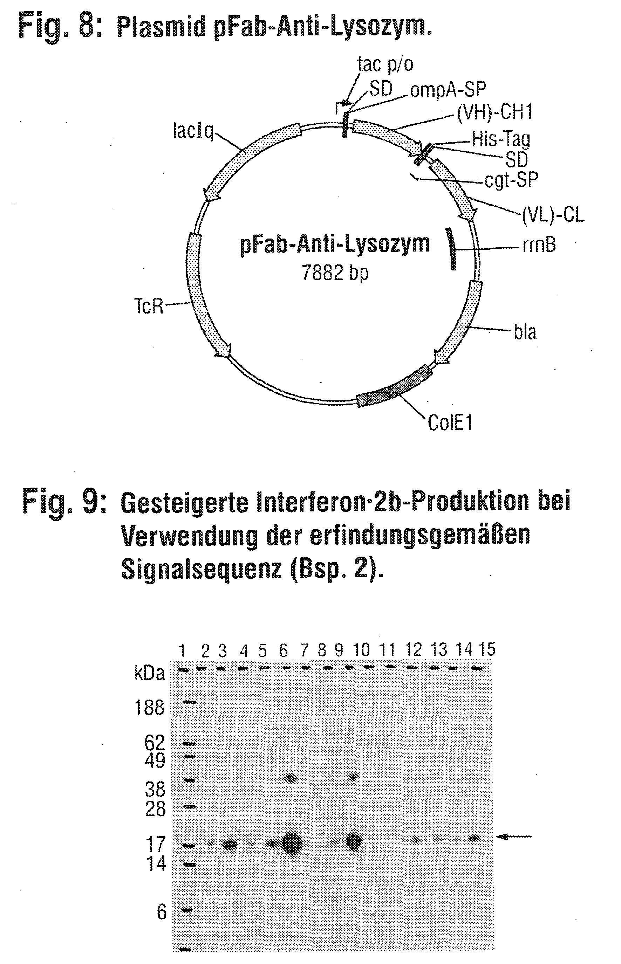

Fig. 9 zeigt einen Immunoblot aus diesem Beispiel. In Tabelle 1 sind die quantifizierten Ausbeuten an Interferonα2b zusammengefasst:

**Tabelle 1: Ausbeuten an Interferonα2b erhalten nach 24, 48 oder 72 h mit verschiedenen Plasmiden, die sich jeweils durch die verwendete Signalsequenz unterscheiden.**

| Plasmid | Signalsequenz | Anzucht (h) | Ausbeute Interferonα2b (mg/l) |
|---|---|---|---|
| pBaBIFN1 | AFA | 24 | 4 |
| pBaBIFN1 | AFA | 48 | 25 |
| pBaBIFN1 | AFA | 72 | 156 |
| pKPIFN | cgt | 24 | 4 |
| pKPIFN | cgt | 48 | 21 |
| pKPIFN | cgt | 72 | 95 |
| pKP651 | phoA | 24 | 0 |
| pKP651 | phoA | 48 | 0 |
| pKP651 | phoA | 72 | 22 |
| pKP652 | ompA | 24 | 4 |
| pKP652 | ompA | 48 | 18 |
| pKP652 | ompA | 72 | 41 |

Dieses Ergebnis zeigt eindeutig, dass die erfindungsgemäße Signalsequenz SEQ ID NO 2 den anderen Signalsequenzen überlegen ist, was die Ausbeute und Sekretion des zu produzierenden Proteins betrifft.

### Beispiel 3: Erzeugung eines verbesserten CGTAse-Produktions-Plasmides durch die Insertion der erfindungsgemäßen Signalsequenz

Das Plasmid pCGT (s. Beispiel 1) trägt das Gen für eine CGTase in frame fusioniert an die Signalsequenz für die CGTase. Diese Signalsequenz wurde nun durch die erfindungsgemäße Signalsequenz ersetzt.

Das 8448 bp große Plasmid wurde dazu mit den Restriktionsenzymen SspI und BglII in einem Partialverdau nach dem Fachmann bekannten Methoden geschnitten. Das 8119 bp große Fragment wurde isoliert und mit Klenow-Enzym behandelt, wodurch die Enden geglättet wurden. Das 329 bp große Fragment, welches die CGTase-Signalsequenz und etwa 150 bp der 5'-Endes des CGTase-Gens enthält, wurde entfernt.
Folgendes 329 bp große DNS-Fragment, welches zu oben genanntem Fragment hinsichtlich Sequenz identisch ist, außer, dass die CGTase Signalsequenz durch die erfindungsgemäße Signalsequenz ersetzt wurde, wurde durch Gensynthese hergestellt:

Dieses DNS-Fragment wurde mit dem isolierten 8119 bp großen Fragment nach einer dem Fachmann bekannten Methode ligiert. Das dadurch entstandene Plasmid pCM703AFA wurde durch Sequenzierung auf Richtigkeit der Sequenz überprüft.

### Beispiel 4: Verbesserung der CGTase-Produktion durch Verwendung der erfindungsgemäßen Signalsequenz

Plasmid pCM703AFA und Plasmid pCGT (siehe Beispiel 3) wurden in folgende Stämme durch Transformation nach gängigen Methoden (z. B. mittels CaCl₂-Transformation) eingeführt:
- K802 = CGSC***** 5610: Yang et al., 1998
- WCM105: herstellbar gemäß EP0338410B1
Selektion auf plasmidhaltige Stämme erfolgte mittels Ampicillin (100 mg/L).
Dadurch wurden folgende erfindungsgemäße Stämme erhalten:
- WCM105/pCM703AFA
- K802/pCM703AFA
Und folgende Kontrollstämme:
- WCM105/pCGT
- K802/pCGT
Diese Stämme wurden zur Produktion einer Cyclodextrin-Glycosyl-Transferase eingesetzt und werden in 10 ml in LB-Medium mit 1 % Glucose und 100 mg/L Ampicillin bei 30°C angezogen. Bei OD 0,5 wird die Produktion der Cyclodextrin-Glycosyl-Transferase durch Zugabe von IPTG (Isopropylthiogalactosid) ad 0,5 mM induziert.
Im Überstand der Anzuchten der Stämme wurde die Ausbeute an Cyclodextrin-Glycosyl-Transferase durch folgenden Aktivitätstest bestimmt:
Testpuffer: 5 mM Tris-HCl-Puffer > pH 6,5, 5 mM CaSO₄ . 2 H₂0
Substrat: 10 %ige Noreduxlösung in Test-Puffer (pH 6,5)
Testansatz: 1 ml Substratlösung + 1 ml abzentrifugierter Kulturüberstand (5 Min., 12 000 rpm) + 3 ml Methanol
Reaktionstemperatur: 40°C

### Enzymtest:

◇ Vortemperieren der Lösungen (ca. 5 Min. bei 40°C)
◇ Zugabe der Enzymlösung zur Substratlösung; rasches Mischen (Whirl-Mixer)
◇ Inkubation für 3 Min. bei 40°C
◇ Stoppen der Enzymreaktion durch Methanol-Zugabe; rasches Mischen (Whirl-Mixer)
◇ Abkühlen des Ansatzes auf Eis (ca. 5 Min.)
◇ Abzentrifugieren (5 Min., 12 000 rpm) und Abpipettieren des klaren Überstandes
◇ Analyse des produzierten CDs mittels HPLC
Enzymaktivität: A = G*V1*V2/(t*MG) (Units/ml)
A = Aktivität
G = Gehalt an CD in mg/l = Testansatz: Flächeneinheiten x 10⁴ / Standardlösung (10 mg/ml)/ Flächeneinheiten
V1 = Verdünnungsfaktor / Testansatz (→ 5)
V2 = Verdünnungsfaktor / Enzymlösung
t = Reaktionszeit in Min. (→ 3)
MG = Molekulargewicht in g/mol (CD → 973)
1 Unit = 1 µMol Produkt / Min.

Tabelle 2 zeigt die gesteigerte spezifische Cyclodextrin-Glycosyltransferase-Ausbeute der erfindungsgemäßen Stämme.

**Tabelle 2: Ausbeute an Cyclodextrin-Glycosyltransferase in verschiedenen Stämmen.**

| Stamm | Produzierte Cyclodextrin-Glycosyltransferase (U/ml) |
|---|---|
| | |
| WCM105/pCGT | 99 |
| WCM105/pCM703AFA | 158 |
| K802/pCGT | 67 |
| K802/pCM703AFA | 81 |

### Beispiel 5: Verbesserung der Hirudin-Produktion durch Verwendung der erfindungsgemäßen Signalsequenz

Das in Patent EP0448093B1 beschriebene Plasmid pCMT203 wurde dahingehend verändert, dass die verwendete Signalsequenz gegen die erfindungsgemäße Signalsequenz AFA ausgetauscht wurde. Dieser Austausch erfolgte in zu Beispiel 1 und 3 analoger Weise. Das entstandene Plasmid wurde pCMT203AFA genannt.
pCMT203 und pCMT203AFA wurden in Stamm WCM105 (herstellbar gemäß EP0338410B1) durch Transformation nach gängigen Methoden (z. B. mittels CaCl₂-Transformation) eingeführt.

Selektion auf plasmidhaltige Stämme erfolgte mittels Ampicillin (100 mg/L).
Folgende Stämme wurden erhalten:
- WCM105/pCMT203
- WCM105/pCMT203AFA
Beide Stämme wurden, wie in EP0448093B1 beschrieben, in einem 10 1 Fermenter kultiviert und das entstandene Hirudin, wie in EP0448093B1 beschrieben, 45 h nach Zugabe von IPTG quantifiziert.
Die Ergebnisse in Tabelle 3 zeigen, dass die Verwendung der erfindungsgemäßen Signalsequenz zu erhöhten Ausbeuten an Hirudin führt.

**Tabelle 3: Ausbeute an Hirudin (in AT-U/ml und g/L) in 10 l Fermentationen 45 h nach Induktion mit IPTG in verschiedenen Stämmen.**

| Stamm | Hirudin (AT-U/ml) | Hirudin (g/L) |
|---|---|---|
| | | |
| WCM105/pCMT203 | 42000 | 2,63 |
| WCM105/pCMT203AFA | 55400 | 3,47 |

### Beispiel 6: Verbesserung der Produktion eines funktionellen Fab-Antikörperfragmentes durch Verwendung der erfindungsgemäßen Signalsequenz

Das vorliegende Beispiel beschreibt die verbesserte Produktion eines Fab-Fragments des gut charakterisierten Anti-Lysozym-Antikörpers D1.3.
Als Ausgangsvektor für die Klonierung und Expression der Gene des Anti-Lysozym-Fab-Fragments diente das Plasmid pJF118ut (s. Beispiel 1). In dieses Plasmid wurden in zwei aufeinanderfolgenden Schritten die beiden Leserahmen für die schwere Kette (V_{H}-C_{H}1-Domänen) bzw. für die leichte Kette (V_{L}-C_{L}-Domänen) des Anti-Lysozym-Fab-Fragments jeweils inklusive einer Signalsequenz kloniert.
Dazu wurde wie folgt vorgegangen:
Das DNS-Fragment mit der SEQ ID NO 12 (schwere Kette) wurde mittels Gensynthese hergestellt und umfasst eine Genfusion bestehend aus der Signalsequenz des ompA-Gens von *E. coli* und dem Leserahmen für die schwere Kette (V_{H}-CH1) des Fab-Fragments. An diesen Leserahmen schließen direkt sechs Histidin-Codons an und bilden somit den C-Terminus des Fusionsproteins. Über diesen His-Tag ist später eine einfache Aufreinigung des vollständig assemblierten Fab-Fragments mittels Affinitätschromatographie möglich. Dieses DNS-Fragment wurde mit den Restriktionsenzymen EcoRI und PstI geschnitten und mit dem Expressionsvektor pJF118ut, der mit denselben Restriktionsenzymen geschnitten worden war, ligiert. Das aus dieser Klonierung resultierende Plasmid, bei dem die Expression des Gens für die schwere Kette unter der Kontrolle des tac-Promotors liegt, wurde mit pHC-Anti-Lysozym bezeichnet.

Das DNS-Fragment mit der SEQ ID NO 13 (leichte Kette) wurde ebenfalls mittels Gensynthese hergestellt und umfasst eine Genfusion bestehend aus einer DNA-Sequenz codierend für das in SEQ ID NO 3 beschriebene Signalpeptid einer CGTase (fett markiert in SEQ ID NO 7) und dem Leserahmen für die leichte Kette (V_{L}-C_{L}) des Fab-Fragments. Dieses DNS-Fragment wurde zunächst mit dem Restriktionsenzym PstI geschnitten und anschließend mit dem Vektor pHC-Anti-Lysozym, der mit demselben Restriktionsenzym geschnittenen worden war, ligiert. Das daraus resultierende Plasmid wurde mit pFab-Anti-Lysozym (Fig. 9) bezeichnet. Auf diese Weise wurde ein artifizielles Operon bestehend aus den jeweiligen Leserahmen für die schwere und die leichte Kette erzeugt, welches unter der Kontrolle des tac-Promotors steht. Damit ist durch Zugabe eines Induktors (z. B. IPTG) eine synchrone Expression der beiden Gene möglich.

Für die Herstellung der erfindungsgemäßen Plasmide pFab-Anti-LysozymVLAFA und pFab-Anti-LysozymVHAFA wurde analog, wie in Beispiel 1 beschrieben, entweder die Signalsequenz für die leichte Kette (pFab-Anti-LysozymVLAFA) oder die Signalsequenz für die schwere Kette (pFab-Anti-LysozymVHAFA) gegen die erfindungsgemäße Signalsequenz SEQ ID NO 2 ausgetauscht.

Für die Herstellung des Anti-Lysozym-Fab-Fragments wurde der Stamm WCM105 (s. Beispiel 4) mit den Plasmiden pFab-Anti-Lysozym, pFab-Anti-LysozymVLAFA bzw. pFab-Anti-LysozymVHAFA nach der CaCl₂-Methode transformiert. Die Selektion auf plasmidhaltige Zellen erfolgte mittels Ampicillin (100 mg/l).

Die Produktion des Anti-Lysozym-Fab-Fragments erfolgte im 10 1-Maßstab. Die Produktion wurde in 10 1 Rührkesselfermentern durchgeführt.
Der mit 6 1 des Mediums FM4 (1,5 g/l KH₂PO₄; 5 g/l (NH₄)₂SO₄; 0,3 g/l MgSO₄ x 7 H₂O; 0,05 g/l CaCl₂ x 2 H₂O, 0,075 g/l FeSO₄ x 7 H₂O; 1 g/l Na₃Citrat x 2 H₂O; 0,5 g/l NaCl); 1 ml/l Spurenelementlösung (0,15 g/l Na₂MoO₄ x 2 H₂O; 2,5 g/l Na₃BO₃; 0,7 g/l CoCl₂ x 6 H₂O; 0,25 g/l CuSO₄ x 5 H₂O; 1,6 g/l MnCl₂ x 4 H₂O; 0,3 g/l ZnSO₄ x 7 H₂O); 5 mg/l Vitamin B₁; 3 g/l Phyton; 1,5 g/l Hefeextrakt; 10 g/l Glukose; 100 mg/l Ampicillin befüllte Fermenter wurde im Verhältnis 1:10 mit einer Vorkultur, die über Nacht im gleichen Medium kultiviert wurde, angeimpft. Während der Fermentation wurde eine Temperatur von 30°C eingestellt und der pH-Wert durch Zudosierung von NH₄OH bzw. H₃PO₄ bei einem Wert von 7,0 konstant gehalten. Glukose wurde über die Fermentation hinweg zudosiert, wobei eine maximale Glukose-Konzentration im Medium von < 10 g/l angestrebt wurde. Die Induktion der Expression erfolgte durch Zugabe von Isopropyl-β-D-thiogalactopyranosid (IPTG) ad 0,1 mM am Ende der logarithmischen Wachstumsphase.

Nach 72 h Fermentation wurden Proben entnommen, die Zellen durch Zentrifugation vom Kulturmedium abgetrennt.
Die Reinigung des Anti-Lysozym-Fab-Fragments aus den Kulturüberständen erfolgte mittels Affinitätschromatographie, wie in Skerra (1994, Gene 141, 79 - 84) beschrieben.

Die Quantifizierung sowie die Bestimmung der Aktivität des gereinigten Anti-Lysozym-Fab-Fragments erfolgte über einen ELISA-Test mit Lysozym als Antigen (Skerra, 1994, Gene 141, 79 - 84).

In Tabelle 4 sind die Ausbeuten an funktionellem Anti-Lysozym-Fab-Fragment aufgelistet, die aus jeweils 20 ml Kulturüberstand nach 72 h Fermentation isoliert werden konnten.

**Tabelle 4: Anti-Lysozym-Fab-Fragment-Ausbeuten im Kulturüberstand nach 72 h Fermentation**

| Stamm | aus 20 ml Überstand gereinigtes Anti-Lysozym-Fab-Fragment [mg] | Anti-Lysozym-Fab-Fragment-Ausbeute [g/l] im Kulturüberstand (hochgerechnet) |
|---|---|---|
| WCM105/ pFab-Anti-Lysozym | 25 | 1,25 |
| WCM105/ pFab-Anti-LysozymVLAFA | 30 | 1,5 |
| WCM105/ pFab-Anti-LysozymVHAFA | 33 | 1,65 |

## Patentansprüche

1. Signalpeptid mit einer Schnittstelle zu einem rekombinanten Protein, welches **dadurch gekennzeichnet ist, dass** es eine Aminosäuresequenz MKRNRFFNTSAAIAISIALQIFFPSASAFA (SEQ ID NO 1) oder eine Aminosäuresequenz, bei der im Vergleich zu SEQ ID NO 1 eine bis zehn, bevorzugt eine bis fünf und insbesondere bevorzugt eine bis drei Aminosäuren mit Ausnahme der drei letzten Aminosäuren vor der Schnittstelle verändert sind, hat und seine drei letzten Aminosäuren vor der Schnittstelle Alanin-Phenylalanin-Alanin (AFA) sind.

2. DNS-Sequenz kodierend für ein Signalpeptid gemäß Anspruch 1.

3. Expressionskonstrukt, umfassend ein Expressionssignal, eine DNS-Sequenz gemäß Anspruch 2 sowie ein in frame verknüpftes rekombinantes Gen kodierend für ein rekombinantes Protein.

4. Plasmid, enthaltend eine DNS-Sequenz gemäß Anspruch 2 oder ein Expressionskonstrukt gemäß Anspruch 3.

5. Gentechnisch veränderte Mikroorganismenzelle, enthaltend ein Expressionskonstrukt gemäß Anspruch 3 oder ein Plasmid gemäß Anspruch 4.

6. Mikroorganismenzelle gemäß Anspruch 5, **dadurch gekennzeichnet, dass** es sich um eine Zelle eines Bakterienstammes der Familie der Enterobacteriaceae, bevorzugt um eine Zelle eines Stammes der Art Escherichia coli handelt.

7. Verfahren zur fermentativen Herstellung eines rekombinanten Proteins, **dadurch gekennzeichnet, dass** eine Mikroorganismenzelle gemäß Anspruch 5 oder 6 in einem Fermentationsmedium kultiviert wird, die Mikroorganismenzelle das rekombinante Protein in Form einer Signalpeptid-Protein-Fusion produziert, wobei das Signalpeptid ein Signalpeptid gemäß Anspruch 1 ist und das Signalpeptid bei der Sekretion der Signalpeptid-Protein-Fusion über die Cytoplasma-Membran in das Periplasma an der Schnittstelle zwischen Signalpeptid und dem rekombinanten Protein abgespalten wird und das gewünschte rekombinante Protein im Periplasma oder dem Fermentationsmedium erhalten wird und das rekombinante Protein nach der Fermentation aufgereinigt wird.

8. Verfahren nach Anspruch 7, **dadurch gekennzeichnet, dass** das rekombinante Protein ein Protein ist, welches Verwendung in technischen Ansätzen findet oder welches als Pharmawirkstoff eingesetzt wird.

## Claims

1. Signal peptide with a cleavage site to a recombinant protein, which is **characterized in that** it has an amino acid sequence MKRNRFFNTSAAIAISIALQIFFPSASAFA (SEQ ID No. 1) or an amino acid sequence wherein, compared to SEQ ID No. 1, one to ten, preferably one to five and particularly preferably one to three amino acids, with the exception of the last three amino acids before the cleavage site, are altered, and the last three amino acids before the cleavage site are alanine-phenylalanine-alanine (AFA).

2. DNA sequence coding for a signal peptide according to Claim 1.

3. Expression construct, comprising an expression signal, a DNA sequence according to Claim 2 and an in-frame linked recombinant gene coding for a recombinant protein.

4. Plasmid containing a DNA sequence according to Claim 2 or an expression construct according to Claim 3.

5. Genetically modified microorganism cell containing an expression construct according to Claim 3 or a plasmid according to Claim 4.

6. Microorganism cell according to Claim 5, **characterized in that** it is a cell of a bacterial strain of the family Entero-bacteriaceae, preferably a cell of a strain of the species Escherichia coli.

7. Process for the fermentative production of a recombinant protein, **characterized in that** a microorganism cell according to Claim 5 or 6 is cultured in a fermentation medium, the microorganism cell produces the recombinant protein in the form of a signal peptide-protein fusion product, wherein the signal peptide is a signal peptide according to Claim 1 and on secretion of the signal peptide-protein fusion product through the cytoplasmic membrane into the periplasm, the signal peptide is cleaved off at the cleavage site between signal peptide and the recombinant protein and the desired recombinant protein is obtained in the periplasm or the fermentation medium and the recombinant protein is purified after the fermentation.

8. Process according to Claim 7, **characterized in that** the recombinant protein is a protein which is used in industrial systems or which is used as a pharmaceutical active substance.

## Revendications

1. Peptide signal comportant un site de clivage conduisant à une protéine recombinante, qui est **caractérisé en ce qu'**il a une séquence d'acides aminés MKRNRFFNTSAAIAISIALQIFFPSASAFA (SEQ ID N° 1) ou une séquence d'acides aminés dans laquelle, par comparaison avec SEQ ID N° 1, un à dix, de préférence un à cinq et d'une manière particulièrement préférée un à trois acides aminés, à l'exception des trois derniers acides aminés avant le site de clivage, sont modifiés, et ses trois derniers acides aminés avant le site de clivage sont alanine-phénylalanine-alanine (AFA).

2. Séquence d'ADN codant pour un peptide signal selon la revendication 1.

3. Construction d'expression comprenant un signal d'expression, une séquence d'ADN selon la revendication 2, ainsi qu'un gène recombinant, lié dans le cadre, codant pour une protéine recombinante.

4. Plasmide contenant une séquence d'ADN selon la revendication 2 ou une construction d'expression selon la revendication 3.

5. Cellule de micro-organisme génétiquement modifiée, contenant une construction d'expression selon la revendication 3 ou un plasmide selon la revendication 4.

6. Cellule de micro-organisme selon la revendication 5, **caractérisée en ce qu'**il s'agit d'une cellule d'une souche bactérienne de la famille des Enterobacteriaceae, de préférence d'une cellule d'une souche de l'espèce Escherichia coli.

7. Procédé de préparation par fermentation d'une protéine recombinante, **caractérisé en ce qu'**une cellule de micro-organisme selon la revendication 5 ou 6 est cultivée dans un milieu de fermentation, la cellule de micro-organisme produit la protéine recombinante sous forme d'une fusion peptide signal-protéine, le peptide signal étant un peptide signal selon la revendication 1, et le peptide signal étant clivé lors de la sécrétion de la fusion peptide signal-protéine, en passant par la membrane cytoplasmique pour pénétrer dans le périplasme, au niveau du site de clivage entre le peptide signal et la protéine recombinante, et la protéine recombinante souhaitée étant obtenue dans le périplasme ou dans le milieu de fermentation, et la protéine recombinante étant purifiée après la fermentation.

8. Procédé selon la revendication 7, **caractérisé en ce que** la protéine recombinante est une protéine qui trouve une utilisation dans des applications techniques, ou est utilisée en tant que principe actif pharmaceutique.
